# EUROPEAN PATENT APPLICATION

(11) **EP 4 530 291 A1**
(43) Date of publication of application: **02.04.2025**
(21) Application number: 23812102.4
(22) Date of filing: 23.05.2023
(51) Int. Cl.: C07K 14/005, C12N 15/70

(54) **PLATFORM FOR EXPRESSING PROTEIN OF INTEREST USING VIRAL NUCLEOCAPSID**

(30) Priority: 23.05.2022 KR 20220062789
(71) Applicant: Vaxdigm Co., Ltd., Seoul 06097 (KR); University-Industry Foundation, Yonsei University, Seoul 03722 (KR)
(72) Inventor: SOHN, Myung Hyun, Seoul 02878 (KR); KIM, Do Da, Siheung-si, Gyeonggi-do 15030 (KR); PARK, Seong Hyun, Incheon 21093 (KR); KIM, Sung Jae, Seoul 06337 (KR); KIM, Seung Hoo, Seoul 02145 (KR)
(74) Representative: Algemeen Octrooi- en Merkenbureau B.V.
(86) International application number: PCT/KR2023/006958
(87) International publication number: WO 2023/229328

(57) **Abstract**

A target protein expression vector of the present invention includes a polynucleotide that encodes a viral nucleocapsid as a fusion partner of a target protein. In addition, a method of producing a target protein of the present invention includes constructing a target protein expression vector, including polynucleotides encoding a target protein, and a viral nucleocapsid as a fusion partner of the target protein; preparing a transformant by introducing the target protein expression vector into a host cell; and culturing the transformant.

## Description

### [Technical Field]

The present invention relates to a target protein expression platform using a viral nucleocapsid. More particularly, the present invention relates to an expression vector that can efficiently express a target protein, particularly, a difficult-to-express protein expressed in an insoluble form in *E. coli,* using a viral nucleocapsid as a fusion partner, transformed host cells, and a method of producing a target protein.

### [Background Art]

The core of modern biotechnology is the production of recombinant proteins, and the most important thing is to produce recombinant proteins in large quantities while maintaining their intrinsic activity. The recombinant proteins produced thereby are very important for the production and recovery of foreign proteins, crystallization for functional studies, and industrialization.

To date, many studies on recombinant protein production using *Escherichia coli* (*E. coli*) have been conducted, which is because *E. coli* has advantages of easy manipulation, short growth time, stable expression, low cost, and easy scale change. However, since foreign recombinant proteins produced in *E. coli* have a lower expression level, or no appropriate "post-translational chaperons" or "posttranslational processing," the disadvantage of the production of the recombinant protein is that it forms an insoluble protein inclusion body (Francois Baneyx, Recombinant protein expression in Escherichia coli, Current Opinion in Biotechnology (1999), 10:411-421).

To solve the above problems, methods of expressing highly soluble proteins such as MBP, NusA, SUMO, thioredoxin or GST by fusion with a recombinant protein have been developed (Dominic Esposito and Deb K Chatterjee, Enhancement of soluble protein expression through the use of fusion tags, Current Opinion in Biotechnology (2006), 17:353-358), but these methods also have technical limitations, so the development of new expression technologies is required.

### [Disclosure]

### [Technical Problem]

The present invention is directed to providing an expression vector that can efficiently express a target protein, particularly, a difficult-to-express protein expressed in an insoluble form in *Escherichia coli* (*E. coli*) in host cells, particularly, *E. coli.*

The present invention is also directed to providing a host cell that is transformed with the expression vector to realize efficient expression of the target protein.

The present invention is also directed to providing a method of efficiently producing the target protein using the expression vector and/or the host cells.

### [Technical Solution]

One aspect of the present invention provides a target protein expression vector, which includes a polynucleotide encoding a viral nucleocapsid as a fusion partner of a target protein.

Another aspect of the present invention provides a host cell transformed with the target protein expression vector.

Still another aspect of the present invention provides a method of producing a target protein, which includes constructing a target protein expression vector, including polynucleotides encoding a target protein and a viral nucleocapsid as a fusion partner of the target protein; preparing a transformant by introducing the target protein expression vector to a host cell; and culturing the transformant.

### [Advantageous Effects]

According to the present invention, a target protein, particularly, a difficult-to-express protein that is expressed in an insoluble form in *Escherichia coli (E. coli),* can be efficiently expressed in host cells, particularly, *E. coli.*

### [Description of Drawings]

FIG. 1 shows the SDS-PAGE result that confirms the expression of a viral nucleocapsid (NC) with a His-tag in *Escherichia coli (E. coli)* to investigate its potential as a fusion partner.
FIG. 2 shows the SDS-PAGE result that confirms the expression of an NC in *E. coli* (expression without a His-tag) to investigate its potential as a fusion partner.
FIG. 3 shows the SDS-PAGE result that confirms the expression of GFP as a target protein after introducing an expression vector according to one embodiment of the present invention into *E. coil.*
FIG. 4 shows the SDS-PAGE result that confirms the expression of GFP as a target protein when introducing an expression vector according to one embodiment of the present invention into *E. coli* and treating the *E. coli* with different IPTG concentrations.
FIG. 5 shows the result of measuring the fluorescence signal of GFP as a target protein using a microplate reader when introducing an expression vector according to one embodiment of the present invention into *E. coli* and treating the *E. coli* with different IPTG concentrations.
FIG. 6 shows the SDS-PAGE result that confirms the expression of IFNβ as a target protein after introducing an expression vector according to one embodiment of the present invention into *E. coli.*
FIG. 7 shows the SDS-PAGE result that confirms the expression of IFNβ in *E*. *coli* without a fusion partner.
FIG. 8 shows the result of an IFNβ response assay by purifying IFNβ as a target protein according to the experiment of FIG. 6 through Ni-affinity chromatography and performing serial dilution.
FIG. 9 shows the amino acid sequences of mutant NCs used in an experiment with a wild-type NC and the interaction thereof with a zinc ion.
FIG. 10 shows the SDS-PAGE result that confirms the expression of a fusion protein when each NC of FIG. 9 is applied.
FIG. 11 shows the result of introducing an expression vector according to one embodiment of the present invention into *E. coil,* inducing the expression of GFP as a target protein, which is purified by zinc affinity chromatography, and analyzed through SDS-PAGE and the measurement of a fluorescence signal of GFP.

### [Modes of the Invention]

According to one embodiment of the present invention, as a vector that can efficiently express a target protein in heterologous or homologous host cells, a target protein expression vector that includes a polynucleotide encoding a viral nucleocapsid as a fusion partner of a target protein is provided.

The term "target protein" used herein refers to a protein that is intended to be produced in large quantities by one of ordinary skill in the art, and encompasses all proteins that can be expressed in host cells by inserting a polynucleotide encoding the protein into a recombinant vector.

In the present invention, the target protein may be one or more selected from the group consisting of an antigen, an antibody, a cell receptor, an enzyme, a structural protein, serum, and a cell protein, but the present invention is not limited thereto.

The term "expression vector" used herein refers to a vector for introducing a polynucleotide encoding a polypeptide of interest into heterologous or homologous host cells and expressing the same, and a cyclic or linear polynucleotide, e.g., a DNA or RNA molecule. An expression vector may include a promoter and/or a terminator sequence, and may also include components necessary for use as a vector, such as a replication origin, a selection marker, and a polyadenylation signal and the like.

The expression vector of the present invention may be a plasmid, a viral vector, a phage particle or a genomic insert, and may be replicated independently of the genome of a host cell or integrated into the genome of a host cell after being introduced into the host cell.

The expression vector of the present invention may include a polynucleotide encoding a target protein, or may be used later by including a polynucleotide encoding a target protein. Particularly, in the latter case, preferably, a restriction enzyme recognition site usually called a multi-cloning site (MCS) as a component that can easily include a target protein-encoding polynucleotide may be included.

The present invention is based on a new research result in that, when a viral nucleocapsid is applied as a fusion partner of a target protein, the target protein, for example, a protein expressed in an insoluble form when a fusion partner is not used or another conventional fusion partner is used, can be efficiently expressed in a soluble form.

Particularly, the fusion partner used in the present invention has an advantage of minimized interference with a target protein due to a very small size (short-length peptide).

In addition, according to the present invention, there is an advantage of using an intrinsic zinc-binding domain of a viral nucleocapsid. For example, using the zinc-binding domain of the viral nucleocapsid, for example, using a zinc affinity purification method, a fusion protein may be easily purified without a separate component for purification, such as a His-tag.

In the present invention, the viral nucleocapsid may be a conventionally known viral nucleocapsid, and may be a fragment or variant of the viral nucleocapsid as long as it acts as a fusion partner of the target protein and maintains the activity of the present invention, that is, the activity to express the target protein in a soluble form, particularly, in *Escherichia coli (E. coli).*

In the present invention, the viral nucleocapsid may be a retrovirus, for example, a nucleocapsid of a retrovirus such as HIV-1, SIV, or MuLV.

In the present invention, the viral nucleocapsid is preferably a nucleocapsid of a human immunodeficiency virus (HIV).

In the present invention, the viral nucleocapsid may include an amino acid sequence having 50% or more, preferably 60% or more, more preferably 70% or more, more preferably 80% or more, more preferably 90% or more, more preferably 95% or more, more preferably 96% or more, more preferably 97% or more, more preferably 98% or more, or more preferably 99% or more sequence identity with the amino acid sequence of SEQ ID NO: 1, or the amino acid sequence of SEQ ID NO: 1. Preferably, the viral nucleocapsid includes an amino acid sequence satisfying the above sequence identity while maintaining amino acids 14, 17, 22, and 27, more preferably, amino acids 14, 17, 22, 27, 35, 38, 43, and 48, and more preferably, amino acids 6, 9, 10, 13, 14, 17, 19, 22, 25, 27, 28, 31, 32, 33, 35, 37, 38, 40, 43, 46, 48, and 51 in SEQ ID NO: 1.

In the present invention, the polynucleotide encoding the viral nucleocapsid may include a base sequence having 50% or more, preferably 60% or more, more preferably 70% or more, more preferably 80% or more, more preferably 90% or more, more preferably 95% or more, more preferably 96% or more, more preferably 97% or more, more preferably 98% or more, or more preferably 99% or more sequence identity with the amino acid sequence of SEQ ID NO: 2 or 3, or the base sequence of SEQ ID NO: 2 or 3. Preferably, the polynucleotide encoding the viral nucleocapsid includes a base sequence that encodes an amino acid sequence maintaining amino acids at positions 14, 17, 22, and 27, more preferably, amino acids 14, 17, 22, 27, 35, 38, 43, and 48, and more preferably, amino acids 6, 9, 10, 13, 14, 17, 19, 22, 25, 27, 28, 31, 32, 33, 35, 37, 38, 40, 43, 46, 48, and 51 in SEQ ID NO: 1, and satisfies the above sequence identity.

In the specification, the sequence identity refers to sequence identity between an amino acid sequence and a reference amino acid sequence, or between a nucleotide sequence and a reference nucleotide sequence. The sequence identity may be determined by comparing the positions of respective sequences that can be aligned for comparison. When the positions of the comparative sequences are occupied by the same amino acid or base, these molecules are the same at these positions. The degree of identity between amino acid or nucleotide sequences is a function of the number of identical amino acids or nucleotides at a position shared by each amino acid or nucleotide sequence. For example, identity between two sequences may be calculated using various alignment algorithms and/or programs, including FASTA or BLAST.

The expression vector of the present invention may be used to express a target protein in cells selected from all organisms including prokaryotes and eukaryotes, preferably, to express a target protein in microorganisms of the genus *Escherichia,* and more preferably, to express a target protein in *Escherichia coli.*

The expression vector of the present invention may further include other components, for example, a cloning site such as a restriction enzyme recognition site, e.g., a multi-cloning site (MCS) for inserting a target protein-encoding polynucleotide; a linker sequence for providing a spacing between a target protein and a fusion partner; a tag sequence, e.g., a His-tag sequence; and/or a protease recognition site-encoding sequence for cleaving a part of the expressed fusion protein. Each component may be arranged in various ways as needed, and may also be included in various numbers.

In an exemplary embodiment, the expression vector of the present invention is configured such that a viral nucleocapsid is linked to the N-terminus of the target protein. As a specific example of the embodiment, the expression vector of the present invention includes a construct in which a promoter, a viral nucleocapsid-encoding site, a target protein-encoding site or cloning site (e.g., a restriction enzyme recognition site such as an MCS) for inserting a target protein-encoding polynucleotide, and a terminator are operably linked sequentially in a 5' to 3' direction.

In another exemplary embodiment, the expression vector of the present invention is configured such that a viral nucleocapsid is linked to the C-terminus of the target protein. As a specific example of the embodiment, the expression vector of the present invention includes a construct in which a promoter, a target protein-encoding site or cloning site (e.g., a restriction enzyme recognition site such as an MCS) for inserting a target protein-encoding polynucleotide, a viral nucleocapsid-encoding site, and a terminator are operably linked sequentially in a 5' to 3' direction.

In still another exemplary embodiment, the expression vector of the present invention is configured to be applied by allowing a user to select whether a fusion partner is linked to the N-terminus of the target protein or whether a fusion partner is linked to the C-terminus of the target protein. As a specific example of the embodiment, the expression vector of the present invention includes a construct in which a promoter, a first cloning site for inserting a target protein-encoding polynucleotide, a viral nucleocapsid-encoding site, a second cloning site for inserting a target protein-encoding polynucleotide, and a terminator are operably linked sequentially in a 5' to 3' direction.

The expression vector of the present invention may include a T7 promoter and a T7 terminator such that the transcription of target protein- and fusion partner-encoding DNAs are regulated by a bacteriophage T7 system. Accordingly, it is possible to produce the target protein more effectively in *E. coli.*

The expression vector of the present invention may be constructed using a conventional recombination method based on a conventional expression vector.

According to another embodiment of the present invention, a host cell transformed by the target protein expression vector of the present invention is provided.

In the present invention, the host cell may be selected from cells of all organisms including prokaryotes and eukaryotes, preferably, cells of microorganisms of the genus *Escherichia,* and more preferably, cells of *E. coli.*

The term "transformation" or "introduction" used herein refers to introduction of a polynucleotide into a host so that the polynucleotide can be replicated as an extrachromosomal factor or through completion of chromosomal integration. A transformation method with the expression vector according to the present invention may include electroporation, a calcium phosphate (CaPO₄) method, a calcium chloride (CaCl₂) method, microinjection, a polyethylene glycol (PEG) method, a DEAE-dextran method, a cationic liposome method, or a lithium citrate-DMSO method, but the present invention is not limited thereto.

According to still another embodiment of the present invention, as a method that can efficiently produce a target protein in heterologous or homologous host cells, a method of producing a target protein, which includes constructing a target protein expression vector, including polynucleotides encoding a target protein and a viral nucleocapsid as a fusion partner of the target protein; preparing a transformant by introducing the target protein expression vector to a host cell; and culturing the transformant, is provided.

Here, for specific details such as the matters related to the target protein expression vector and the introduction of the target protein expression vector, the details described above may be referred to.

For culturing a transformant, any culture method known to be suitable for a host cell may be used. For example, when the host cell is *E. coli,* a method of culturing the cell in a Luria-Bertani (LB) medium at 16 to 37 °C may be used. When the expression vector includes a component of an antibiotic selection marker (e.g., a gene resistant to an antibiotic, such as ampicillin), here, the proliferation of *E. coli* including the expression vector may be preferentially induced by culturing the cells by adding the corresponding antibiotic (e.g., ampicillin) to the medium. In addition, when the expression vector includes components that regulate the transcription and/or translation of target protein-encoding DNA, for example, lac operon-related components, the expression of the target protein may be induced by adding a related material such as IPTG.

The following examples are provided to help understanding of the present invention and do not limit the scope of the claims of the present invention.

### [Examples]

### Preparation Example 1

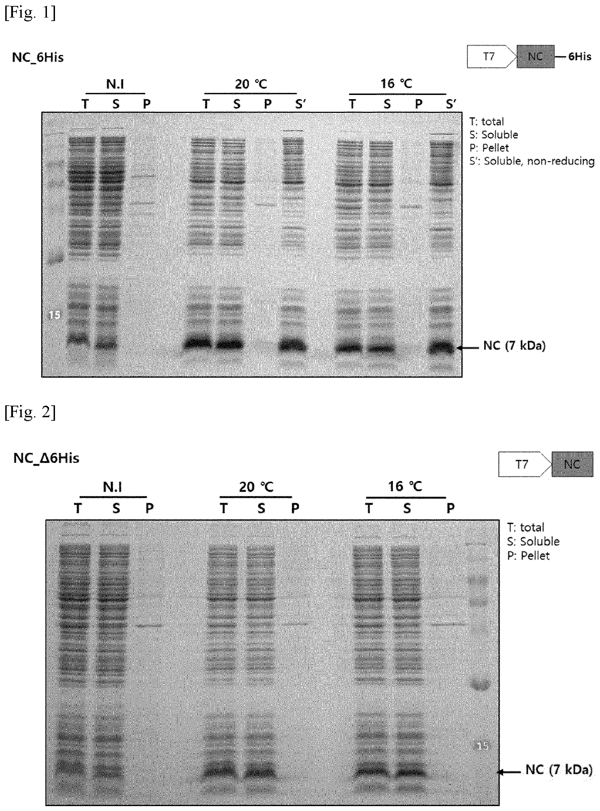

To investigate the possibility of using a viral nucleocapsid (NC) as a fusion partner, a vector for expressing an NC of HIV virus was constructed (FIG. 1). Here, six histidines were additionally applied for purification.

The vector was constructed based on a pGEMEX-1 (Promega) vector designed for target protein expression to be regulated by a T7 promoter, and the sequence of SEQ ID NO: 2 was used as a sequence encoding the NC.

### Preparation Example 2

Compared to Preparation Example 1, an expression vector without six histidines was constructed (FIG. 2).

### Example 1

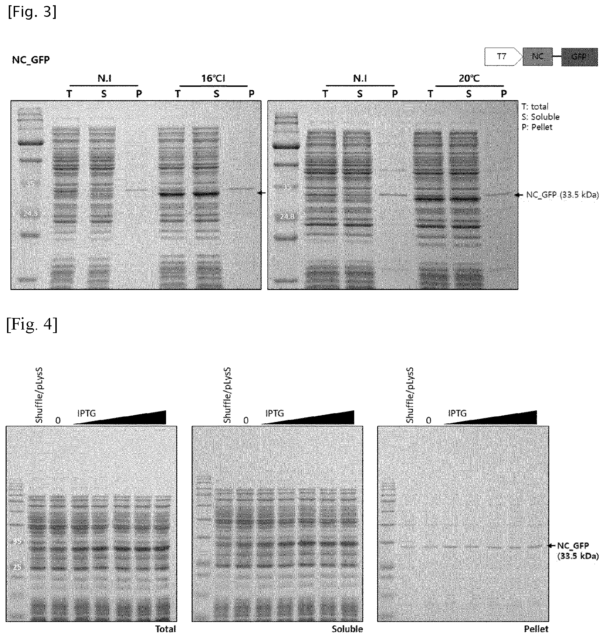

A vector for expressing a target protein, green fluorescent protein (GFP), in the form of linking an NC as a fusion partner to the N-terminus of the protein was constructed (FIG. 3).

The vector was constructed in the same manner as in Preparation Example 2, except that an NC-encoding sequence (SEQ ID NO: 2) and a target protein-encoding sequence were inserted into an MCS of a pGEMEX-1 (Promega) vector.

The NC- and GFP-encoding sequence regions in the constructed vector are the same as SEQ ID NO: 4, and the amino acid sequence of an NC+GFP fusion protein expected to be expressed is the same as SEQ ID NO: 5.

### Example 2

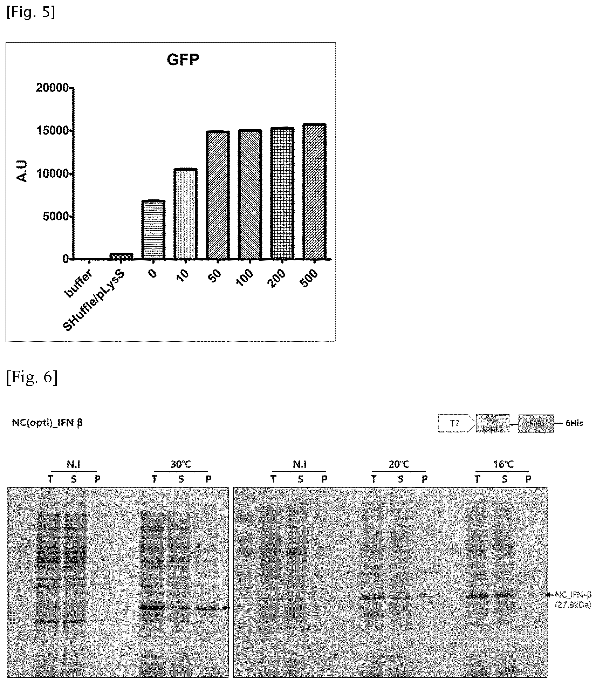

A vector for expressing a target protein, human-derived interferon-beta (IFNβ), in the form of linking NC as a fusion partner to the N-terminus was constructed (FIG. 6). Here, six histidines were additionally applied for purification.

The vector was constructed in the same manner as in Example 1, except that an *E. coli* codon optimization sequence, which is the sequence of SEQ ID NO: 3, was used as a sequence encoding an NC.

The NC- and IFNβ-encoding sequence regions in the constructed vector are the same as SEQ ID NO: 6, and the amino acid sequence of an NC+IFNβ fusion protein expected to be expressed is the same as SEQ ID NO: 7.

### Comparative Example 1

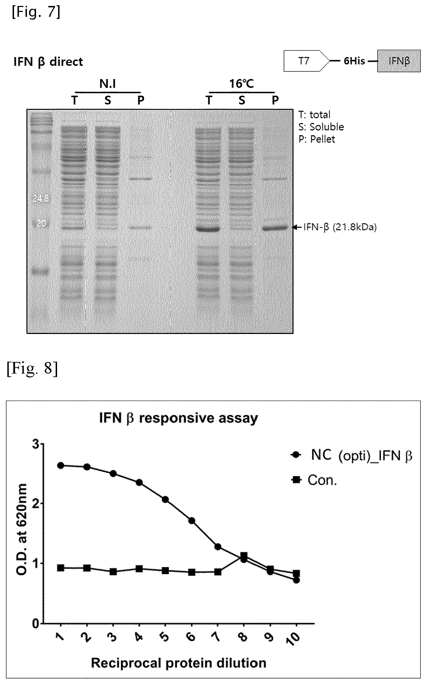

A vector for expressing a target protein, human-derived IFNβ, was constructed in the same manner as in Example 2, except that six histidines were linked to the N-terminus instead of an NC as a fusion partner (FIG. 7).

### Experimental Example 1

*E. coli* Shuffle/T7/pLysS was transformed with each of the vectors of Preparation Example 1, Preparation Example 2, Example 1, Example 2 and Comparative Example 1, and then incubated. The Shuffle/T7/pLysS was prepared by extracting a pLysS plasmid from BL21 star (DE3) pLysS One Shot^{™} Invitrogen^{™} competent *E. coli* through Mini-prep, introducing pLysS into the Shuffle T7(NEB) competent *E. coli,* and selecting the resulting transformant using chloramphenicol (CM), which is an antibiotic marker included in pLysS. All the transformed *E. coli* was cultured in an LB medium containing 50 µg/mL ampicillin and 34 µg/mL chloramphenicol. A culture temperature was set to 16 to 37 °C. When the OD600 value of *E. coli* was 0.5 or more, for activation of a T7 promoter, IPTG was added at a level of 0 µM to 1 mM, and then incubated at 30 °C for 3 hours or at 16 to 20 °C for approximately 16 hours to sufficiently produce proteins. The sufficiently incubated *E. coli* was centrifuged to remove a supernatant and then stored. Afterward, 0.3 mL of PBS was added to the *E. coli* harvest corresponding to 5 mL of the LB medium, and then the harvested *E. coli* was lysed by sonication, thereby preparing a lysate. In addition, the lysate was centrifuged and divided into a soluble fraction and a pellet fraction, and then the total lysate, the soluble fraction, and the pellet fraction were analyzed through SDS-PAGE.

First, as a result of inducing NC expression by introducing each of the vectors of Preparation Examples 1 and 2 into *E. coli,* as shown in FIGS. 1 and 2, NC was expressed normally, i.e., with an expected size and solubility, and in large quantities at both 16 °C and 20 °C. Particularly, as shown in FIG. 2, even when there was no His-tag, it was expressed almost 100% soluble. This shows that it is possible to use an NC as a desired fusion partner to increase the expression efficiency of a target protein, particularly, to be expressed in a soluble form.

As a result of inducing the expression of the target protein, GFP, by introducing the vector of Example 1 into *E. coli,* as shown in FIG. 3, GFP was expressed normally at both 16 °C and 20 °C. GFP is known to be expressed in an insoluble form in *E*. *coli.* Therefore, this result shows that, according to the method of the present invention, a protein expressed in an insoluble form in *E. coli* can be expressed in a soluble form.

In addition, as a result of investigating GFP expression when the vector of Example 1 is introduced into *E. coli* and then the resulting *E. coli* is treated with different IPTG concentrations, as shown in FIG. 4, it was found that even with a low concentration of IPTG, GFP is efficiently expressed in proportion to the IPTG concentration. As a result of measuring a GFP fluorescence signal of the expressed protein using a microplate reader, as shown in FIG. 5, it was found that the GFP fluorescence signal increases in proportion to the IPTG concentration. This shows that, according to the method of the present invention, a target protein can be efficiently expressed while maintaining its original function.

As a result of inducing the expression of the target protein IFNβ by introducing each of the vectors of Example 2 and Comparative Example 1 into *E. coli,* as shown in FIGS. 6 and 7, Example 2 shows that, although the proportion of insoluble expression at 30 °C was relatively high, a large amount of protein was normally expressed in a soluble form at both 16 °C and 30 °C, whereas Comparative Example 1 shows that most of the protein was expressed in an insoluble form. This shows that, according to the present invention, the proteins expressed in an insoluble form in *E*. *coli* can be expressed in a soluble form.

In addition, as a result of performing an IFNβ response analysis (InvivoGen) experiment by inducing the expression of the target protein IFNβ by introducing the vector of Example 2 into *E. coli,* purifying the IFNβ through Ni-affinity chromatography, and serially diluting the resulting product by 1/2 based on initial 200 nM, as shown in FIG. 8, an IFNβ response was confirmed in the purified product, and this IFNβ response was found to decrease as the dilution factor increased. Here, as a control (Con), a medium was used. This result shows that, according to the method of the present invention, the target protein can be efficiently expressed while maintaining its original function.

### Example 3

A vector for expressing a target protein, a heat-labile enterotoxin B subunit (LTB) of *E. coli,* in the form of linking an NC as a fusion partner to the N-terminus was constructed. Here, six histidines were additionally applied for purification, and the same method as in Example 1 was used.

### Example 4

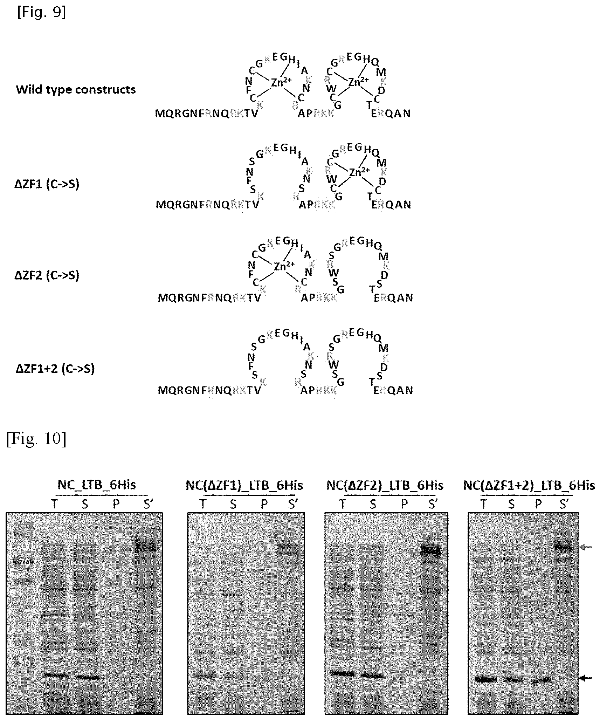

A vector was constructed in the same manner as in Example 3, except that the NC was expressed in the form in which cysteines (C) 14, 17, and 27 in the NC amino acid sequence of SEQ ID NO: 1 were substituted with serine (S) (ΔZF1 of FIG. 9). These residues are residues that interact with a zinc ion in the first zinc-binding domain of the NC along with histidine (H) 22.

### Example 5

A vector was constructed in the same manner as in Example 3, except that the NC was expressed in the form in which cysteines (C) 35, 38, and 48 in the NC amino acid sequence of SEQ ID NO: 1 were substituted with serine (S) (ΔZF2 of FIG. 9). These residues are residues that interact with a zinc ion in the second zinc-binding domain of NC along with histidine (H) 43.

### Example 6

A vector was constructed in the same manner as in Example 3, except that the NC was expressed in the form in which cysteines (C) 14, 17, 27, 35, 38, and 48 in the NC amino acid sequence of SEQ ID NO: 1 were substituted with serine (S) (ΔZF1+2 of FIG. 9).

### Experimental Example 2

After transforming each of the vectors of Examples 3 to 6 into *E. coli* Shuffle/T7/pLysS in the same manner as in Experimental Example 1, the production of a fusion protein was induced, and each produced fusion protein was analyzed by SDS-PAGE.

As a result, as shown in FIG. 10, while Examples 3 and 5 show that almost all the fusion protein was normally expressed in a soluble form, Examples 4 and 6 show that the proportion of fusion proteins expressed in a soluble form was reduced by approximately 50%. This shows that the residues interacting with zinc ions in the first zinc-binding domain of an NC are important in the soluble expression of the target protein.

### Experimental Example 3

After transforming the vector of Example 1 into *E. coli* Shuffle/T7/pLysS in the same manner as in Experimental Example 1, the production of a fusion protein was induced, which was purified by zinc affinity chromatography, and analyzed by SDS-PAGE and the measurement of a GFP fluorescence signal (using a microplate reader).

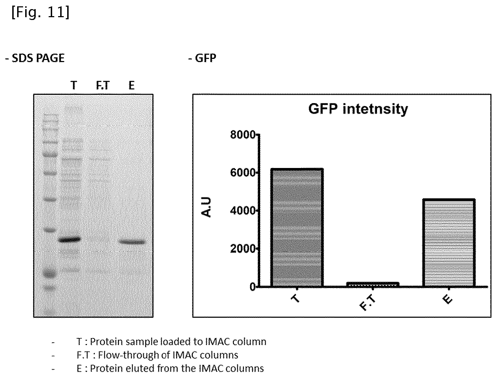

As a result, as shown in FIG. 11, it was shown that the fusion protein can be purified with a considerably high recovery rate through zinc affinity chromatography. This shows that efficient purification is possible using the zinc-binding domain of an NC without a separate purification tag such as a His-tag.

In the above, the present invention was described with reference to embodiments. It will be understood by those of ordinary skill in the art that the present invention can be implemented in modified forms without departing from the essential features of the present invention. Therefore, the disclosed embodiments should be considered from a descriptive perspective, rather than a restrictive perspective. The scope of the present invention is shown in the claims rather than the foregoing description, and all differences within the equivalent range thereto will be construed as being included in the present invention.

### <Sequence listing>

SEQ ID NO: 1
   Sequence name : Amino acid sequence of HIV nucleocapsid
   Molecule type : AA
   Organism : human immunodeficiency virus
   Sequence : 
QRGNFRNQRKTVKCFNCGKEGHIAKNCRAPRKKGCWRCGREGHQM
KDCTERQAN
SEQ ID NO: 2
   Sequence name : Nucleotide sequence encoding HIV nucleocapsid
   Molecule type : DNA
   Organism : human immunodeficiency virus
   Sequence : 
CAGCGGGGAAACTTCAGGAACCAGAGAAAAACTGTGAAGTGCTTC
AATTGCGGAAAGGAGGGCCACATCGCTAAGAACTGCCGCGCCCCCAGAA
AGAAAGGCTGCTGGAGATGCGGCAGAGAGGGCCACCAGATGAAGGACTG
CACAGAGAGACAGGCAAAC
SEQ ID NO: 3
   Sequence name : Nucleotide sequence encoding HIV nucleocapsid
   Molecule type : DNA
   Organism : synthetic construct
   Sequence : 
CAGCGTGGTAACTTCCGTAACCAGCGTAAAACCGTTAAATGCTTCA
ACTGCGGCAAAGAAGGCCACATCGCGAAAAACTGCCGTGCGCCGCGTAA
 
AAAAGGCTGCTGGCGTTGCGGCCGTGAAGGCCACCAGATGAAAGATTGC
ACCGAACGTCAGGCGAAC
SEQ ID NO: 4
   Sequence name : Nucleotide sequence encoding fusion protein of NC and GFP
   Molecule type : DNA
   Organism : synthetic construct
   Sequence : 
AGTAAAGGAGAAGAACTTTTCACTGGAGTTGTCCCAATTCTTGTTG
AATTAGATGGTGATGTTAATGGGCACAAATTTTCTGTCAGTGGAGAGGGTG
AAGGTGATGCAACATACGGAAAACTTACCCTTAAATTTATTTGCACTACTG
GAAAACTACCTGTTCCATGGCCAACACTTGTCACTACTTTCTCTTATGGTG
TTCAATGCTTTTCAAGATACCCAGATCATATGAAACGGCATGACTTTTTCAA
GAGTGCCATGCCCGAAGGTTATGTACAGGAAAGAACTATATTTTTCAAAGA
TGACGGGAACTACAAGACACGTGCTGAAGTCAAGTTTGAAGGTGATACCC
TTGTTAATAGAATCGAGTTAAAAGGTATTGATTTTAAAGAAGATGGAAACA
TTCTTGGACACAAATTGGAATACAACTATAACTCACACAATGTATACATCAT
GGCAGACAAACAAAAGAATGGAATCAAAGTTAACTTCAAAATTAGACAC
AACATTGAAGATGGAAGCGTTCAACTAGCAGACCATTATCAACAAAATAC
TCCAATTGGCGATGGCCCTGTCCTTTTACCAGACAACCATTACCTGTCCAC
ACAATCTGCCCTTTCGAAAGATCCCAACGAAAAGAGAGACCACATGGTCC
TTCTTGAGTTTGTAACAGCTGCTGGGATTACACATGGCATGGATGAACTAT
ACAAA
SEQ ID NO: 5
   Sequence name : Amino acid sequence of fusion protein of NC and GFP
   Molecule type : AA
   Organism : synthetic construct
   Sequence : 
SKGEELFTGVVPILVELDGDVNGHKFSVSGEGEGDATYGKLTLKFICT
TGKLPVPWPTLVTTFSYGVQCFSRYPDHMKRHDFFKSAMPEGYVQERTIFFK
DDGNYKTRAEVKFEGDTLVNRIELKGIDFKEDGNILGHKLEYNYNSHNVYIM
 
ADKQKNGIKVNFKIRHNIEDGSVQLADHYQQNTPIGDGPVLLPDNHYLSTQS
ALSKDPNEKRDHMVLLEFVTAAGITHGMDELYK
SEQ ID NO: 6
   Sequence name : Nucleotide sequence encoding fusion protein of NC and IFNβ
   Molecule type : DNA
   Organism : synthetic construct
   Sequence : 
AAATCTTACAACCTGCTGGGCTTCCTGCAGCGTTCCTCCAACTTCC
AGTGCCAGAAGCTGCTGTGGCAGCTGAACGGTCGCCTGGAATACTGCCTG
AAAGATCGTATGAACTTCGATATCCCGGAAGAAATCAAACAGTTGCAGCA
GTTCCAGAAAGAAGATGCGGCGTTAACCATCTACGAAATGCTGCAAAACA
TTTTCGCTATCTTCCGTCAGGATTCCAGCTCCACCGGCTGGAACGAAACCA
TCGTTGAAAACCTTCTGGCGAACGTTTATCACCAGATCAATCATCTGAAAA
CCGTGCTGGAAGAGAAACTGGAAAAAGAAGACTTCACCCGTGGTAAACT
GATGTCCTCGCTGCACCTGAAGCGTTACTACGGTCGTATCCTGCATTATCTG
AAAGCGAAAGAGTACAGCCACTGCGCTTGGACCATTGTTCGTGTTGAAAT
TCTGCGCAATTTCTACTTCATCAACCGTCTGACCGGTTACCTGCGTAAC
SEQ ID NO: 7
   Sequence name : Amino acid sequence of fusion protein of NC and IFNβ
   Molecule type : AA
   Organism : synthetic construct
   Sequence :

KSYNLLGFLQRSSNFQCQKLLWQLNGRLEYCLKDRMNFDIPEEIKQL
QQFQKEDAALTIYEMLQNIFAIFRQDSSSTGWNETIVENLLANVYHQINHLKT
VLEEKLEKEDFTRGKLMSSLHLKRYYGRILHYLKAKEYSHCAWTIVRVEILR
NFYFINRLTGYLRN

## Claims

1. A target protein expression vector, comprising a polynucleotide encoding a viral nucleocapsid as a fusion partner of a target protein.

2. The vector of claim 1, wherein the target protein is one or more selected from the group consisting of an antigen, an antibody, a cell receptor, an enzyme, a structural protein, serum, and a cell protein.

3. The vector of claim 1, wherein the viral nucleocapsid is a nucleocapsid of human immunodeficiency virus (HIV).

4. The vector of claim 1, wherein the viral nucleocapsid includes the amino acid sequence of SEQ ID NO: 1.

5. The vector of claim 1, wherein the vector is for expressing a target protein in *Escherichia coli (E. coli).*

6. A host cell transformed with the target protein expression vector of any one of claims 1 to 5.

7. A method of producing a target protein, comprising:
constructing a target protein expression vector, including polynucleotides encoding a target protein and a viral nucleocapsid as a fusion partner of the target protein;
preparing a transformant by introducing the target protein expression vector into a host cell; and
culturing the transformant.

8. The method of claim 7, wherein the target protein is one or more selected from the group consisting of an antigen, an antibody, a cell receptor, an enzyme, a structural protein, serum, and a cell protein.

9. The method of claim 7, wherein the viral nucleocapsid is a nucleocapsid of human immunodeficiency virus (HIV).

10. The method of claim 7, wherein the viral nucleocapsid includes the amino acid sequence of SEQ ID NO: 1.

11. The method of claim 7, wherein the host cell is *Escherichia coli (E. coli).*
